# EUROPEAN PATENT SPECIFICATION

(11) **EP 3 829 599 B1**
(45) Date of publication and mention of the grant of the patent: **04.12.2024**
(21) Application number: 19768909.4
(22) Date of filing: 02.08.2019
(51) Int. Cl.: A61K 31/734, A61K 31/728, A61K 31/80, A61K 33/00, A61K 33/10, A61K 36/82, A61P 27/04, A61P 1/04, A61K 9/00, A61K 9/08, A61K 9/20, A61K 47/36

(54) **COMBINATION FOR USE IN THE TREATMENT OF EXTRA-OESOPHAGEAL SYMPTOMS OF GASTRIC REFLUX**
KOMBINATION ZUR VERWENDUNG BEI DER BEHANDLUNG VON EXTRAÖSOPHAGEALEN SYMPTOMEN VON GASTRISCHEM REFLUX
COMBINAISON DESTINEE A ETRE UTILISEE DANS LE TRAITEMENT DE SYMPTOMES EXTRA- SOPHAGIENS DU REFLUX GASTRIQUE

(30) Priority: 02.08.2018 IT 201800007771
(43) Date of publication of application: 09.06.2021
(73) Proprietor: Drugs Minerals and Generics Italia S.R.L. In Forma Abbreviata D.M.G. Italia S.R.L., 00071 Pomezia (RM) (IT)
(72) Inventor: MERCURI, Luigi, 00071 Pomezia (RM) (IT)
(74) Representative: Benedetto, Marco
(86) International application number: PCT/IB2019/056598
(87) International publication number: WO 2020/026205

(56) References cited:
- WO-A1-2009/064617
- WO-A1-95/01795
- ANONYMOUS: "Gastrotuss Anti-Reflux Chewable Tablets", 1 May 2017 (2017-05-01), XP055646917, Retrieved from the Internet <URL:http://dl.vitamed.pl/file/1265/9e14/3889/9bb2/7870/bcc4/c087/9e83/FG_GASTROTUSS%20COMPRESSEpdf.pdf> [retrieved on 20191127]
- IANNELLA GIANNICOLA ET AL: "Investigation of pepsin in tears of children with laryngopharyngeal reflux disease", INTERNATIONAL JOURNAL OF PEDIATRIC OTORHINOLARYNGOLOGY, ELSEVIER, AMSTERDAM, NL, vol. 79, no. 12, 30 October 2015 (2015-10-30), pages 2312 - 2315, XP029368847, ISSN: 0165-5876, DOI: 10.1016/J.IJPORL.2015.10.034

## Description

The present invention regards a combination (C) comprising:(I) a composition (C1) in form of eye wash comprising:
(i) a mixture (M1) comprising, or alternatively, consisting of:
   (i-a) an alginic acid, or a salt thereof,
   (i-b) a hyaluronic acid, or a salt thereof, and, optionally,
(ii) at least one technological additive and/or at least one pharmaceutical or food grade excipient; and
(II) a composition (C2) in form of tablet comprising:
(iii) a mixture (M2) comprising, or alternatively, consisting of:
   (iii-a) an alginic acid or a salt thereof of an alkaline metal or of an alkaline earth metal;
   (iii-b) a bicarbonate salt of an alkaline metal or alkaline earth and/or a carbonate salt of an alkaline metal or alkaline earth;
   (iii-c) a simethicone and/or dimethicone; and, optionally,
(iv) at least one technological additive and/or at least one pharmaceutical or food grade excipient;
said combination (C) being for use in a method for the preventive or curative treatment of symptoms or diseases of the eyeball and periocular area, wherein said symptoms or diseases relate to, or derive from extraoesophageal reflux or laryngopharyngeal reflux (LPR) or from the backflow of the gastric content or of the acid gastric vapours from the stomach into at least one extraoesophageal area.

The present invention further regards a kit comprising said combination (C) for said use, wherein said kit comprises at least one housing in which the composition (C1) in form of eye wash is received and in which the composition (C2) in form of tablet is received.

In the context of the present invention, the expression "combination" is used to indicate two or more compositions together as defined in the context of the present invention.

In the context of the present invention, the expression "composition" is used to indicate a pharmaceutical composition or medical device composition according to the European regulation on medical devices [UE 2017/745 -(MDR), Directive 93/42/CEE -(MDD)] or dietary supplement product or a food product or a food for special medical purpose.

In the context of the present invention the following nosological entities are defined: gastroesophageal reflux, laryngopharyngeal reflux (alternatively defined extraoesophageal reflux) and non-erosive reflux.

Gastroesophageal reflux or the gastroesophageal reflux disease (abbreviated as GERD) is a para-physiological characterised by the backflow of the acid gastric content or acid gastric vapours from the stomach to the oesophagus (from now henceforth, simply, gastric content) for example comprising pepsin, hydrochloric acid, gastric juices, duodenal juices and acid gastric vapours.

The cardia is the anatomic communication region between the oesophagus and stomach, normally located in the abdomen between 2 and 4 cm below the diaphragm. In the cardia there occurs the transition between the oesophageal mucosa and the gastric mucosa.

Right upstream of the cardia, the circular muscle fibres of the oesophagus acquire a sphincter action, in the sense that they remain contracted in the rest condition, while they relax during eructation, the descent of the food into the stomach and its backflow when vomiting; in the other stages of the digestive process, instead, this functional sphincter should normally remain shut and contracted, so as to prevent the acid gastric content of the stomach from flowing back into the oesophagus, irritating the inner walls thereof. The sphincter we are talking about is known as the cardiac sphincter, gastroesophageal sphincter, lower oesophageal sphincter (LES) or cardia.

Cardia incapacity, more or less temporary, such to make the reverse movement of the gastric content possible, occurs in patients suffering from gastroesophageal reflux symptoms or diseases (GERD). In patients suffering from GERD, part of the gastric content flows back from the stomach to the oesophagus, which is not provided with systems for protection for example against hydrochloric acid or against gastric juices or against acid gastric vapours produced by the stomach. Gastric juice is a secretion produced by the internal mucosa of the stomach containing mucus, salts, water, digestive enzymes (such as for example pepsin) and hydrochloric acid. The pH of the gastric content or gastric juice is very low, but for example variable between 1 and 2 in any case.

Thus, in patients suffering from GERD, the backflow - even at very small amounts - of the gastric content from the stomach to the oesophagus causes the irritation of the epithelium of the oesophagus, which causes a burning feeling at the retrosternal position and pains upon deglutition, as well as an increase of caries (due to the corrosion of the teeth enamel caused by the gastric acids), retching after taking food, acidity feeling at the upper part of the oesophagus and in the pharynx (particularly frequent when one lies horizontally after eating, due to the fact that this facilitates the backflow process of the gastric content into the oesophagus).

Gastroesophageal reflux is very common, temporary at times, sometimes a symptom of a much more severe disease. The most common causes can be obesity, diabetes mellitus, conditions of increased gastric secretion, pregnancy, smoking, alcohol, hiatal hernia (congenital condition in which the position of the oesophagus hiatus, the opening of the oesophagus in the stomach, is in an unusual position that facilitates the backflow of the gastric content).

Laryngopharyngeal reflux (abbreviated as LPR from the English acronym Laryngopharyngeal Reflux) or laryngopharyngeal reflux disease or, alternatively, extraoesophageal reflux are the various terms used for identifying the symptoms or diseases caused by the backflow of gastric acid, for example the backflow of pepsin, hydrochloric acid, acid gastric vapours or gastric and/or duodenal juices, from the stomach to the extraoesophageal areas.

In the context of the present invention, the upper respiratory tract, the eyeball (or the eye), the periocular area, the lacrimal apparatus and the ear apparatus, the Eustachian tube and the middle ear are referred to as extraoesophageal areas.

In the context of the present invention, the nasal cavity, the paranasal sinuses, the oral cavity, the pharynx, the epiglottis and the larynx are referred to as upper respiratory tract.

In the context of the present invention, the region between the eye or regarding the eye contour, such as for example the conjunctiva and the lacrimal apparatus, are referred to as the periocular area.

The conjunctiva is a mucosa membrane, which covers the eyeball and the inner part of the eyelids.

The lacrimal apparatus is the set of secretory system of the lacrimal film, which includes the lacrimal gland, and of the apparatus that allows the outflow thereof. The apparatus that allows the outflow thereof includes the lacrimal ways, consisting of: lacrimal points, lacrimal sac and lacrimal channels or lacrimal ducts or nasolacrimal duct.

In other words, when the gastric acid flows back from the stomach, irritative conditions that can involve, for example, the eyeball (or the eye) and the periocular area (e.g. the conjunctiva and the lacrimal apparatus) may occur at the extraoesophageal areas level.

In the context of the present invention, the terms "laryngopharyngeal reflux" (LPR) and "extraoesophageal reflux" will be used interchangeably to indicate what has been defined above.

The main symptoms of LPR (or extraoesophageal reflux) are, for example, dry cough, dysphonia, globus pharyngis, different from the classical GERD "heartburn". Contrary to what occurs in classical gastroesophageal reflux, where the reflux events mainly occur at night, in case of LPR it has been proven that the events occur throughout the 24 hrs.

Despite laryngopharyngeal reflux (LPR) or extraoesophageal reflux having been initially considered as an extension of the gastroesophageal reflux disease (GERD), today it is considered as an independent disease in that it is capable of locally determining disease forms.

The two diseases, GERD and LPR, can coexist or manifest themselves separately.

Should GERD and LPR coexist, patients with GERD, besides the diseases in the oesophagus region, may also suffer from diseases (for example ailment or non-severe illness) or symptoms (for example manifestation of a pathological condition or even a combination of events through which the disease is manifested) in one or more extraoesophageal areas.

Should GERD and LPR not coexist, diseases or symptoms felt in one or more extraoesophageal areas may also occur in subjects not affected by GERD, following the backflow of the gastric content from the stomach into the extraoesophageal area.

In light of the above, people suffering from LPR or extraoesophageal reflux can be affected, for example, from the lacrimal dysfunction syndrome (abbreviated as LDS or OSD (Ocular Surface Disease) alternatively defined as dry eye syndrome. As a matter of fact, there has been observed the presence of gastric acid content in the lacrimal secrete of subjects suffering from LPR (Magliulo, G. et al. Medical Hypotheses 2013, 80, 129-130; lannella, G. et al. Int. J. Pediatr. Otorhinolaringol. 2015, 79, 2312-2315). The lacrimal dysfunction syndrome (LDS) is an eye disease which consists in a quantity reduction and/or in a quality alteration of the lacrimal film, which mainly has a function of moistening and protecting the front surface of the eyeball. The lacrimal film has the task of carrying out crucial functions i) nutritional: the tears allow to guarantee a correct oxygen content and nutritional substances for an appropriate turnover of the cells of the ocular surface; ii) anti-microbial: the presence of antibodies and enzymes in the lacrimal film guarantees a defensive action against external attacks; iii) cleaning and iv) lubricating. Thus, the alteration in the quantity/quality of the lacrimal film entails exposing the eyeball to a greater friction (determined by the movement of the eye lids) and a greater risk of infections. The lacrimal dysfunction syndrome is among the most frequent diseases in ophthalmology and the impact thereof is high in the middle age and elderly population.

In the context of the present invention, the terms "lacrimal dysfunction syndrome" (LDS) and "dry eye syndrome" will be used interchangeably to indicate what has been defined above.

Another symptom or disease that may occur in subjects suffering from LPR or extraoesophageal reflux is the inflammation of the eyeball or the inflammation of the periocular area, in particular conjunctivitis, i.e. an inflammation that affects the conjunctiva, causing the reddening of the eyes. Conjunctivitis may affect adults, children and new-borns alike.

Lastly, in the context of the present invention there is defined the nosological entity of the non-erosive reflux or non-erosive reflux disease (abbreviated as NERD), characterised by the presence of gastroesophageal reflux or laryngopharyngeal reflux (LPR) in absence of endoscopic damage visible under gastroscopy, in that the reflux of stomach acid content does not cause oesophagitis.

Frequently, symptoms or diseases in extraoesophageal areas, such as the upper respiratory tract, the eyeball (or the eye), the periocular area (e.g. Conjunctiva and lacrimal apparatus) and the ear apparatus, are analysed, diagnosed and/or treated without considering a possible correlation with the backflow of the gastric acid content from the stomach to the extraoesophageal areas.

Hence, many of said symptoms or diseases in extraoesophageal areas, such as for example lacrimal dysfunction syndrome (LDS), conjunctivitis or ocular or periocular inflammation, are treated using products currently available in the market that do not curatively and/or preventively treat the phenomenon relating to the backflow of the gastric acid content from the stomach to the extraoesophageal areas; such as for example the eyeball and/or the periocular area.

The prior art document WO 2018/100468 A1 reveals an oral composition comprising at least one alginic acid salt, hyaluronic acid, a natural molecule and thickening agents.

The prior art document EP 1 468 677 A2 reveals a medical device in form of syrup containing silicone oils, magnesium alginate, carboxypolymethylene, re-epithelization compounds and sedative compounds.

The Applicant posed the technical problem of providing combinations, compositions, formulations, preparations or mixtures (in short, combinations) for methods for the preventive and/or curative treatment of symptoms or diseases manifested in at least one extraoesophageal area, relating to or deriving from extraoesophageal reflux or LPR, as defined in the context of the present invention, in subjects suffering from gastroesophageal reflux or in subjects not suffering from gastroesophageal reflux.

In particular, the Applicant posed the technical problem of providing combinations for use in methods for the preventive and/or curative treatment of symptoms or diseases of the eyeball and/or periocular area relating to or deriving from said extraoesophageal reflux, such as for example the lacrimal dysfunction syndrome (LDS), conjunctivitis or ocular or periocular inflammation.

Furthermore, the Applicant posed the technical problem of providing combinations for use in said treatment methods that are effective and avoid relapses, that are stable, east to take/apply and that, once administered, are well-tolerated by the majority of subjects and basically without adverse effects.

Lastly, the Applicant posed the technical problem of providing combinations for use in said treatment methods comprising compositions that do not contain steroidal substances and thus without the adverse effects typically observed in subjects treated using steroidal substances, such as for example the increase of the ocular pressure or risk of glaucoma when the combinations of the invention, in particular, for the treatment of symptoms or diseases of the eyeball and/or periocular area.

Following an extensive and deep research and development activity, the Applicant appropriately solved the technical problem outlined above.

The Applicant surprisingly found combinations of two compositions, without steroidal substances, for use in methods for the treatment of symptoms or diseases manifested in at least one extraoesophageal region (e.g. eyeball or periocular area), relating to or deriving from extraoesophageal reflux (or LPR), such as for example, the lacrimal dysfunction syndrome (LDS), conjunctivitis and/or ocular or periocular inflammation that are effective, stable, easy to take/apply, well tolerated by the majority of subjects to whom it is administered and basically without adverse effects.

Forming an object of the present invention is a combination of two compositions for use in a treatment method having the characteristics as defined in the attached claims.

Furthermore, forming an object of the present invention is a kit comprising said combination (C) for said use, wherein said kit comprises at least one housing in which the composition (C1) in form of eye wash is received and in which the composition (C2) in form of tablet is received, having the characteristics as defined in the attached claims.

Preferred embodiments of the present invention will be apparent from the detailed description below and they are indicated in the attached claims.

It is also disclosed a combination (C) comprising:(I) a composition (C1) comprising (i) a mixture (M1), as defined hereinafter, and, optionally, (ii) at least one technological additive and/or at least one pharmaceutical or food grade excipient; and (II) a composition (C2) comprising (iii) a mixture (M2), as defined hereinafter, and, optionally, (iv) at least one technological additive and/or at least one pharmaceutical or food grade excipient (in short the combination (C)); saic combination being for use in the preventive and/or curative treatment of symptoms or diseases of the eyeball and periocular area, relating to or deriving from extraoesophageal reflux, laryngopharyngeal reflux or, generally, from the backflow of the gastric content or acid gastric vapours from the stomach in at least one extraoesophageal area or in more than one extraoesophageal area.

It is also disclosed a combination (C) comprising: (I) a composition (C1) in form of eye wash comprising (i) a mixture (M1), as defined hereinafter, and, optionally, (ii) at least one technological additive and/or at least one pharmaceutical or food grade excipient; and (II) a composition (C2) in form of tablet comprising: (iii) a mixture (M2), as defined hereinafter, and, optionally, (iv) at least one technological additive and/or at least one pharmaceutical or food grade excipient (in short the combination (C));said combination (C) being for use in a method for the preventive and/or curative treatment of symptoms or diseases of the eyeball and periocular area, wherein said symptoms or diseases relating to or deriving from extraoesophageal reflux or laryngopharyngeal reflux (LPR) or from the backflow of the gastric content or of the acid gastric vapours from the stomach into said at least one extraoesophageal area.

Said (ii) and (iv), at least one technological additive and/or pharmaceutical or food grade excipient, can be selected - by way of non-limiting examples and irrespective of each other - from among a preservative, antioxidant, stabiliser, thickener, rheology modifier, biocide, dye, pH buffers and the like.

By way of non-limiting example, said (ii) and (iv) can be selected, irrespectively of each other, from among sorbitol, mannitol, calcium phosphate dibasic anhydrous, flavour, silicon dioxide, corn starch, vegetable magnesium stearate, sucralose, cellulose and derivatives and microcrystalline cellulose.

In particular, the composition (C1) may comprise at least one technological additive and/or excipient known to the man skilled in the art and useable in ophthalmic formulations or for topical ocular use, wherein said formulations can be liquid, semi-solid or solid.

The combination (C) for use according to the attached claims can be for use in human subjects or for veterinarian use, by way of non-limiting example, in pets such as dogs or cats, or in other mammals. Preferably, the combination (C) according to the present invention is for use in humans.

The combination (C) for use according to the attached claims is for use both in diseased subjects (patients who have been diagnosed with a disease) and in healthy subjects (non-diseased subjects).

The combination (C) for use according to the attached claims is for use both in subjects suffering from gastroesophageal reflux disease (GERD), i.e. patients suffering from GERD or in subjects with the typical symptoms and problems relating to GERD, or in subjects not suffering from GERD, i.e. in subjects who have not been diagnosed with GERD or who show the typical symptoms and problems relating to GERD.

Preferably, the combination (C) is for use in subjects suffering from gastroesophageal reflux disease (GERD).

In an embodiment, the combination (C) for use according to the attached claims is for use in the preventive and/or curative treatment of diseases or symptoms that are manifested in the or diseases of the eyeball or periocular area.

In a preferred embodiment, the combination (C) for use according to the attached claims is for use in the preventive and/or curative treatment of symptoms or diseases relating to or deriving from the lacrimal dysfunction syndrome or dry eye syndrome, conjunctivitis of the conjunctiva, conjunctivitis of the cornea, ocular inflammation or periocular inflammation.

In a preferred embodiment, the combination (C) for use according to the attached claims is for use in the simultaneously preventive or curative treatment of one or more first symptoms or disease relating to or deriving from extraoesophageal reflux and, simultaneously, of one or more second symptoms or disease relating to or deriving from extraoesophageal reflux, wherein said one or more first symptoms or diseases are symptoms manifested in the or diseases of the eyeball, of the periocular area and/or of the lacrimal apparatus, and wherein said one or more second symptoms or diseases are not symptoms manifested in the or diseases of the eyeball, of the periocular area and/or of the lacrimal apparatus.

Advantageously, for example, the use of the combination (C) allows the simultaneous preventive or curative treatment of diseases of the eye area (eyeball, periocular area and lacrimal apparatus), such as for example, lacrimal dysfunction syndrome or dry eye syndrome, conjunctivitis of the conjunctiva, conjunctivitis of the cornea, ocular inflammation or periocular inflammation as well as diseases of the upper respiratory tract, such as for example irritations or inflammations of the upper respiratory tract mucosae, relating to or deriving from the extraoesophageal reflux or laryngopharyngeal reflux.

Hence, according to an advantageous aspect, the combination (C) for use according to the attached claims allows the treatment of diseases closest to the main causes (the extraoesophageal reflux or laryngopharyngeal reflux or the backflow of the gastric content or of the gastric acid gastric vapours from the stomach) of such diseases that develop in a subject in a further position with respect to, and due to, such main cause.

To express such concept in an even more concise manner, the combination (C) for use according to the attached claims allows the treatment of a subject both in proximity of the cause or of the source of disease as well as the symptoms triggered by such cause that are manifested in relatively remote extraoesophageal areas (for example at the eye and ear).

Advantageously, the combination (C) for use according to the attached claims allows to carry out said simultaneous treatment of said one or more first symptoms or diseases using smaller doses of at least one, or both, from among the composition (C1) and the composition (C2) of the present invention with respect to the utilised doses of the composition (C1) and/or of the composition (C2) when used singularly. The use of smaller dose of one or both compositions (C1) and (C2) allows, advantageously, to reduce the risk of occurrence of adverse effects.

Alternatively, the combination (C) for use according to the attached claims allows to carry out said simultaneous treatment of said one or more first symptoms or diseases and said one or more second symptoms or diseases using the same doses of the composition (C1) and the composition (C2) of the present invention with respect to the doses used when (C1) and (C2) are used singularly, but offering a treatment of at least one among said first or second symptoms or diseases more effective with respect to when obtained using (C1) and (C2) singularly.

In a preferred embodiment, the combination (C) for use according to the attached claims is for use as coadjuvant for the re-epithelialization processes of a mucosa in at least one extraoesophageal area, preferably of the mucosa of the eyeball or periocular area.

In a preferred embodiment, the combination (C) for use according to the attached claims is for use in subjects with Reflux Symptom Index greater than 13.

There are various methods for the diagnosis of laryngopharyngeal reflux (LPR), both anamnestic and objective or instrumental. Among them, recognised and accepted at international level is the use of a symptom severity score called Reflux Symptom Index (abbreviated as RSI), a symptomatology score which, despite its simplicity, allows an accurate diagnosis of the presence/absence of the laryngopharyngeal reflux disease and the severity of the symptoms.

In a preferred embodiment, the combination (C) for use according to the attached claims is for use in subjects with Reflux Symptom Index greater than 13 and Ocular Surface Disease Index greater than 12, preferably with Ocular Surface Disease Index greater than 22, more preferably with Ocular Surface Disease Index greater than 32.

There are various methods for the diagnosis of lacrimal dysfunction syndrome (LDS), both anamnestic and objective or instrumental. Among them, recognised and accepted at international level is the use of a symptom severity score called *Ocular Surface Disease Index* (abbreviated as OSDI), which allows an accurate diagnosis, and staging of the lacrimal dysfunction syndrome.

In an embodiment, the composition (C1) of the present invention is administered topically and wherein the composition (C2) is administered enterally.

In other words, the composition (C1) comprising (i) the mixture (M1) as described hereinafter and, optionally, (ii) the at least one technological additive and/or at least one pharmaceutical or food grade excipient, is administered topically. The composition (C2) comprising (iii) the mixture (M2) as described hereinafter and, optionally, (iv) at least one technological additive and/or at least one pharmaceutical or food grade excipient, is administered enterally.

Preferably, the composition (C1) is administered in an ocular topical manner, for example on an eyeball and/or periocular area surface, and the composition (C2) is administered enterally, such as for example orally (or gastrointestinal means), rectally and sublingually (or buccal).

Preferably, the composition (C1) is administered topically in the eyeball and/or periocular area surface (ocular topically) and the composition (C2) is administered orally.

In an embodiment, the composition (C1) is administered topically in the eyeball and/or periocular area surface (ocular topically) and the composition (C2) is administered orally.

It should be observed that forming an object of the present invention the combination (C) for use according to the attached claims is for use in the preventive and/or curative treatment of symptoms or diseases relating to or deriving from extraoesophageal reflux or laryngopharyngeal reflux or, generally, from the backflow of the gastric content or acid gastric vapours from the stomach in at least one extraoesophageal region, both when the composition (C1) and the composition (C2) are administered to a subject in any order, both when the composition (C1) and (C2) are administered to a subject in a close sequence over time or in a non-close sequence, both when (C1) and (C2) are administered to a subject with the same frequency or with different frequencies.

According to an embodiment, the composition C1 in form of eye wash could be administered from one to four times (preferably from two to three times) per day, at an amount comprised between one to three drops per eye for each administration. According to another embodiment, the composition C2 in form of tablet could be administered from one to four times (preferably from two to three times) per day, at an amount of one tablet or two tablets for each administration.

Advantageously, the composition (C) according to the present invention allows the curative and/or preventive treatment of the symptoms and the diseases caused by gastroesophageal reflux and that are manifested in the eye and/or in the periocular area without administering substances of the steroidal type, that could cause adverse effects, even severe, and they can deteriorate the gastroesophageal reflux disease, besides causing the increase of ocular pressure and increasing the risk of glaucoma.

Preferably, the composition (C1) according to the present invention does not contain active ingredients of the steroidal type.

The mixture (M1) of the present invention comprises or, alternatively, consists of:
(i-a) an alginic acid, or a salt thereof, and
(i-b) a hyaluronic acid, or a salt thereof.

In a first embodiment, the mixture (M1) consists of: (i-a) an alginic acid, or a salt thereof, and (i-b) a hyaluronic acid or a salt thereof, and the composition (C1) does not contain active ingredients besides those present in said mixture (M1).

In a second embodiment, the mixture (M1) comprises, besides (i-a) an alginic acid, or a salt thereof, and (i-b) a hyaluronic acid or a salt thereof, at least another compound or active ingredient; preferably, said at least another compound or active ingredient is selected from among:
(i-c) extract of green tea *(Camellia sinensis),* preferably glyceric extract of green tea at an amount comprised between 0.1 % and 5% by weight; more preferably at an amount comprised between 0.5% and 3.5% by weight; even more preferably at an amount comprised between 1 % and 2% by weight, with respect to the weight of the composition (C1);
(i-d) rosmarinic acid (3-(3,4-dihydroxyphenyl)-2R-{[3-(3,4-dihydroxyphenyl)prop-2E-enoyl]oxy}propanoic acid), preferably at an amount comprised between 0.01% and 3% by weight; more preferably at an amount comprised between 0.1% and 2% by weight; even more preferably at an amount comprised between 0.5% and 1 % by weight, with respect to the weight of the composition (C1); and
(i-e) ethylenediaminetetraacetic acid (EDTA) or a salt thereof, preferably at an amount comprised between 0.05% and 0.3% by weight; more preferably at an amount comprised between 0.1% and 0.25% by weight; even more preferably at an amount comprised between 0.15% and 0.20% by weight, with respect to the weight of the composition (C1).

Preferably, both in case of the first embodiment and the second embodiment, the composition (C1) comprises (i-a) and (i-b) in the following percentages by weight with respect to the total weight of the composition (C1):
- (i-a) is comprised between 0.05% and 5% by weight; preferably it is comprised between 0.2% and 2% by weight; even more preferably it is comprised between 0.4% and 0.8% by weight, and
- (i-b) is comprised between 0.01 % and 1 % by weight; preferably it is comprised between 0.05% and 0.5% by weight; even more preferably it is comprised between 0.15% and 0.30% by weight.

Preferably, both in the case of the first embodiment and in the second embodiment, the mixture (M1) comprises (i-a) an alginic acid (CAS n 9005-32-7), a colloidal and hydrophilic polysaccharide, having an (average) molecular weight comprised between about 50 kDalton (kDa) and about 800 kDa; preferably comprised between about 100 kDa and about 600 kDa; even more preferably comprised between about 200 kDa and about 400 kDa, preferably at about 240 kDa, wherein kDalton (kDa) corresponds to 1000 Dalton (Da) and 1 Dalton corresponding to 1 unified atomic mass unit (u). Preferably, (i-a) is obtained from marine algae. Preferably, (i-a) it is an alginate salt, more preferably magnesium alginate.

The mixture (M1) according to the present invention comprises (i-b) a hyaluronic acid (CAS N. 9004-61-9) in acid or salified form, for example such as sodium salt.

The hyaluronic acid is a non-sulphated glycosaminoglycan having a non-branched polysaccharide chain deriving from the condensation of disaccharide units in turn formed by residues of glucuronic acid and N-acetylglucosamine bonded to each other, alternatively, by β1→4 and β 1→3 glycosidic bonds.

Preferably, both in the case of the first embodiment and in the second embodiment, the mixture (M1) may comprise (i-b) a hyaluronic acid, or the salts thereof, having a different origin and various molecular weight ranges and it can be linear or branched

Preferably, the mixture (M1) comprises (i-b) a linear or branched hyaluronic acid, or salts thereof, for example sodium salt, with a (average) molecular weight comprised between 200 kDa and 5000 kDa; more preferably it is comprised between 1000 kDa and 3000 kDa; even more preferably it is comprised between 1500 kDa and 2000 kDa.

In a preferred embodiment, the combination (C) comprises the composition (C1) comprising the mixture (M1 a) comprising, or alternatively, consisting of:
- (i-a) magnesium salt of an alginic acid (a magnesium alginate),
- (i-b) sodium salt of a hyaluronic acid (a sodium hyaluronate), and
- (i-c) extract of green tea or Camellia sinensis.

It should be observed that, it is also disclosed the use of (i-a) and (i-b), as defined above, in the treatment of symptoms or diseases relating to or deriving from extraoesophageal reflux, preferably symptoms or diseases manifested in or diseases of the eyeball and/or periocular area, both when (i-a) and (i-b) are administered simultaneously, i.e. Mixed in the same composition (C1), and when (i-a) and (i-b) are administered to a subject separately by means of two separate compositions (C1- a) and (C1-b), in close sequence over time (and for example at the 30-minute time interval) and in any order.

The composition (C1) according to the present invention is in form of eye wash.

In an embodiment, the composition (C1) according to the present invention is in form of ophthalmic solution, namely eye wash, comprising boric acid and/or sodium tetraborate preferably, irrespective of each other, at an amount comprised between 0.1 and 1% by weight on the total weight of the pharmaceutical composition or medical device.

In a particularly preferred embodiment, the combination (C) comprises the composition (C1) in form of ophthalmic solution (. eye wash) wherein (C1) comprises in by weight % per 500 g of total weight of composition (C1):

| Component | % weight/total weight | Weight (g) |
|---|---|---|
| - Magnesium alginate | 0.20 | 1 |
| - Hyaluronic acid | 0.15 | 0.75 |
| - Glyceric extract of green tea (*Camellia senensis*) | 1.00 | 5 |
| and, optionally, excipients such as: | | |
| - Sodium tetraborate | 0.20 | 1 |
| - Boric acid | 0.70 | 3.5 |
| - Sodium chloride | 0.24 | 1.2 |
| - Water | 97.51 | 487.55 |

The composition (C2) of the present invention comprises a mixture (M2) comprising or, alternatively, consisting of:
(iii-a) an alginic acid, or a salt thereof of an alkaline metal or of an alkaline earth metal;
(iii-b) a bicarbonate salt of an alkaline metal or alkaline earth and/or a carbonate salt of an alkaline metal or alkaline earth; and
   - (iii-c) a simethicone and/or a dimethicone.

In the mixture (M2) of the present invention it is observed that:
- (iii-a) the alginic acid, or a salt thereof of an alkaline metal or of an alkaline earth metal, is present, together with (iii-b) and (iii-c), in said mixture at an amount in weight comprised between 20% and 70%; preferably it is present at an amount by weight comprised between 40% and 60%, with respect to the final weight of the mixture (M2);
- (iii-b) the bicarbonate salt of an alkaline metal or of an alkaline earth metal and/or the carbonate salt of an alkaline metal or of an alkaline earth metal, is/are present in said mixture at an amount by weight comprised between 5% and 30%; preferably it/they is/are present at an amount by weight comprised between 10% and 25%, with respect to the final weight of the mixture (M2);
- (iii-c) the simethicone and/or dimethicone, is/are present in said mixture at an amount by weight comprised between 5% and 30%; preferably it/they is/are present at an amount by weight comprised between 10% and 25%, with respect to the to the final weight of the mixture (M2).

In the composition (C2) of the present invention it is observed that:
- (iii-a) the alginic acid, or the salt thereof of an alkaline metal or of an alkaline earth metal, is present, together with (iii-b) and (iii-c), in said composition at an amount in weight comprised between 1% and 30%, preferably between 10% and 20% by weight, with respect to the final weight of the composition;
- (iii-b) the bicarbonate salt is present at an amount by weight comprised between 0.1% and 15%, preferably between 1% and 8% by weight, with respect to the final weight of the composition;
- (iii-c) the simethicone or dimethicone (iii-c) is present at an amount by weight comprised between 0.05% and 15%, preferably between 0.5% and 5% by weight, with respect to the final weight of the composition (C2).

Advantageously, the composition (C2) of the present invention comprises the mixture (M2) of the present invention at a ratio by weight comprised between 1:10 [mixture (M2): composition (C2)] and 1 :2 [mixture (M2): composition (C2)]; preferably at a ratio by weight of about 1 :4 [mixture (M2): composition (C2)] or at a ratio by weight of about 1 :3 [mixture (M2): composition (C2)].

Preferably, in said mixture (M2) said (iii-a) alginic acid is preferably an alginic acid having an average molecular weight comprised between about 50 kDalton (KDa) and about 1000 kDa; more preferably comprised between about 100 kDa and about 800 kDa; more preferably comprised between about 200 kDa and about 600 kDa; more preferably comprised between about 300 kDa and about 400 kDa; wherein kDalton (kDa) corresponds to 1000 Dalton (Da) and 1 Dalton corresponds to 1 unified atomic mass unit (u).

Preferably, (iii-a) is an alginic acid or an alginic acid salt having an average molecular weight of about 240.000 Dalton (unified atomic mass unit, u), i.e. 240 kDa, and (iii-a) is obtained from marine algae.

The alginic acid (iii-a) comprised in the mixture (M2) is in form of an alginate salt of an alkaline metal or of an alkaline earth metal; more preferably, in said mixture (M2) said alginate salt is selected from among the group comprising or, alternatively, consisting of: sodium alginate, potassium alginate, calcium alginate or magnesium alginate. Alginic acid is a polysaccharide consisting of two types of uronic acid: Mannuronic acid unit (M) and Guluronic acid unit (G), which form two types of polymeric segment blocks: M blocks with M-M bonds only; G blocks with G-G bonds only; M&G random blocks with M-G random bonds.

Examples of (iii-a) alginic acid and alginates used in the present invention together with (iii-b) and (iii-c) are represented by:
- alginic acid EP in form of a straw yellow powder with an average grain size of about 80 mesh, a viscosity less than or equal to 50 cPs, an acidity index greater than or equal to 230, carboxylic groups of about 19-25, a pH of about 2-3.5;
- calcium alginate, CAS n.° 90005-35-0, with general formula (C₆ H₇Ca_{1/2}06)ₙ ; structural units 195.16 (theoretical), 219 (average) and macromolecules: 10,000-600,000 (typical average);
- magnesium alginate (KIMICA ALGIN MAG-60, (composition: Guluronic acid of about 65-75% and Mannuronic acid of about 25-35% and a magnesium content of about 5.4-6.6) with a pH value of about 6-9,5, a viscosity (CPS) (LV, 12RPM) 7.5% natural basic solution, 25°C of about 1 ,000-1 ,800.
- potassium alginate, CAS n.° 90005-36-1 , with general formula (C₆H₇KO6)ₙ; structural units 214.22 (theoretical), 238 (average) and macromolecules: 10,000-600,000 (typical average).
- satialgine^{™} S 110 (composition: sodium alginate E401 and saccharose) with a viscosity of about 550- 750 mPa s (1 % sol.) and pH of about 6-8.5 (1% sol.);
- sodium alginate, CAS n.° 90005-38-3, with general formula (C₆H₇Na06)ₙ ; structural units 198,11 (theoretical), 222 (average) and macromolecules: 10,000-600,000 (typical average);

Preferably, in the mixture (M2) (iii-a) there is the alginic acid having CAS no. 9005-32-7.

The mixture (M2) of the present invention comprises, combined with said alginic acid (iii-a), (iii-b) a bicarbonate salt of an alkaline metal or of an alkaline earth metal (b) selected from among the group comprising or, alternatively, consisting of: sodium bicarbonate, potassium bicarbonate, magnesium bicarbonate and calcium bicarbonate; and/or (iii-b) a carbonate salt of an alkaline metal or of an alkaline earth metal (b) selected from among the group comprising or, alternatively, consisting of; sodium carbonate, potassium carbonate, magnesium carbonate and calcium carbonate.

An example of potassium bicarbonate (b) is (E501 ii) with general formula HKCO₃, molecular weight 100, 12 and EINECS n.° 298-14-6 and a *Bulk density* of about 0.99-1.12 g/cm3 and a Tapped density of about 1.14-1.25 g/cm³.

The mixture (M2) of the present invention comprises, in association with said (iii-a) alginic acid, or a salt thereof, and said (iii-b) bicarbonate salt and/or said (iii-b) carbonate salt, (iii-c) a simethicone compound, or (iii-c) a dimethicone compound, or (iii-c) a simethicone compound and a dimethicone compound together. Examples of (iii-c) simeticone to be used in association with (iii-a) and (iii-b), are represented by:
- Simethicone ANTI FOAM 30 MG in form of water-soluble simethicone (aqueous emulsion) with an antifoam activity of 15 seconds max. and an active content comprised between 29% and 32% by weight and a density of about 1 g/cm³ ;
- BC Simethicone Antifoam C100F, this compound consists of a fluid polydimethylsiloxane (PDMS) and a silica aerogel. The silica aerogel activates the antifoam properties of the fluid silicon. This compound has a specific gravity at 25°C of about 0.1; a viscosity at 25°C of about 3000 cP; an efficiency less than 15 seconds; a composition of PDMS of about 92.5-97.5% and silica of about 4-7%;
- Simethicone SILFAR^{®} SE4 in form of an emulsion with a solid content of about 31-34% by weight; an active gradient ("Simethicone", USP) of about 30% by weight, a density at 25°C of about 1 g/ml, a viscosity at 25°C of about 1500-5000 mPas, a pH at 25°C of about 3-5.

Preferably, said (iii-c) simethicone is the simethicone having CAS no. 8050-81-5; or it is a simethicone belonging to the following classes of simethicone: Simethicone 100, Simethicone Emulsion 30%, Simethicone Emulsion, Antifoam C 100, Antifoam 30 PD, Antifoam PD30 S, Antifoam 30 MG.

Examples of (iii-c) of dimethicone are represented by:
- Dimethicone ACESIL 350, CAS n.° 63148-62-9, in liquid form with a kinematic viscosity at 25°C of about 332.5-367.5 mm2/s, a density at 25°C of about 0.9660-0.9720 g/ml, a refractive index at 25°C of about 1.4025-1.4045, chemical name polydimethylsiloxane;
- Dimethicone ABIL^{®} 350, CAS n.° 63148-62-9, in liquid form with a refractive index of about 1.4030-1.4050, a density at 25°C of about 0.95-0.97 g/ml and a viscosity at 25°C of about 332-368 mm2/s;
- PEG-14 Dimethicone ABIL^{®} B 8843 (components: PEG-14 Dimethicone 95% by weight and propylene glycol 5% by weight), CAS n.° 68937-54-2, in liquid form with a refractive index of about 1.4500-1.4600, a density at 25°C of about 1.063-1.077 g/ml and a viscosity at 25°C of about 300-600 mPas; and
- Dimethicone ABIL^{®} WAX 2434 (component: Stearoxy Dimethicone 100%), CAS n.° 68554-53-0, with a refractive index of about 1.4210-1.4270, a density at 50°C of about 0.8600-0.9000 g/ml and a recrystallisation of about 20-30 °C.

Preferred examples of (iii-c) dimethicone are: Cetyl dimethicone, Stearyl dimethicone, Stearoxy dimethicone, Behenoxy dimethicone and all polymers belonging to the group of dimethicone copolyols. Non-limiting examples of Copolyol dimethicone are: Dimethicone PEG-8 Adipate, Dimethicone PEG-8 Benzoate, Dimethicone PEG-7 Phosphate, Dimethicone PEG-10 Phosphate, Dimethicone PEG / PPG- 20/23 Benzoate, Dimethicone PEG / PG-7 Dimethicone, PEG-10 Dimethicone, PEG-9 Dimethicone, PEG- 10 Dimethicone, PEG-12 Dimethicone, PEG-3 Dimethicone, PEG-7 Dimethicone, PEG- 14 Dimethicone, PEG/PPG-8/14 Dimethicone, PEG/PPG-3/10 Dimethicone, PEG/PPG-4/12 Dimethicone, PEG/PPG-6/11 Dimethicone, PEG/PPG-4 Dimethicone, PEG/PPG-15/15 Dimethicone, PEG/PPG-16/2 Dimethicone, PEG/PPG-17/18 Dimethicone, PEG/PPG-18/18 Dimethicone, PEG/PPG-19/ PPG-20/6 dimethicone, PEG/PPG-20/15 dimethicone, PEG/PPG-20/20 dimethicone, PEG/PPG-20/23 dimethicone, PEG/PPG-20/29 dimethicone, PEG/PPG-22/23 dimethicone, PEG/PPG-22/24 dimethicone, PEG/PPG-23/6 dimethicone, PEG/PPG-25/25 dimethicone and PEG/PPG-27/27 dimethicone.

It is also disclosed the use of (iii-a), (iii-b) and (iii-c), as defined above, in the treatment of symptoms or diseases relating to or deriving from extraoesophageal reflux, preferably symptoms manifested in or diseases of the eyeball and/or periocular area, both when (iii-a), (iii-b) and (iii-c) are administered simultaneously, i.e. mixed in the same composition (C2), and when (iii-a), (iii-b) and (iii-c) are administered to a subject separately by means of two separate compositions (C2-a), (C2-b) and (C2-c), in close sequence over time (and for example at the 30-minute time interval) and in any order.

In a preferred embodiment, the mixture (M2) comprises (iii-c) simethicone having CAS no. 8050-81-5.

In a preferred embodiment, the composition (C2) comprises the mixture (M2a) consists of:
- (iii-a) sodium salt of an alginic acid (a magnesium alginate),
- (iii-b) potassium bicarbonate;
- (iii-c) a simethicone and/or a dimethicone.

In a preferred embodiment, the composition (C2), in form of tablet, has the following composition by weight per 100 g of (C2):
- Sorbitol: 40 g
- Magnesium alginate: 17 g
- Mannitol: 14 g
- Calcium phosphate dibasic anhydrous: 5 g
- Potassium bicarbonate: 5 g
- Flavour: 5 g
- Silicon dioxide: 4 g
- Corn starch: 4.4 g
- Simethicone: 3.5 g

Vegetable magnesium stearate 2 g
- Sucralose: 0.1 g

In a preferred embodiment, the combination (C) comprises the composition (C1) comprising the mixture (M1 a) comprising, or alternatively, consisting of:
- (i-a) sodium salt of an alginic acid (a magnesium alginate),
- (i-b) sodium salt of a hyaluronic acid, and
- (i-c) extract of green tea or *Camellia sinensis* ; and, furthermore, the combination (C) comprises the composition (C2) comprising the mixture (M2a) comprising, or alternatively, consisting of:
- (iii-a) sodium salt of an alginic acid (a magnesium alginate),
- (iii-b) potassium bicarbonate;
- (iii-c) a simethicone and/or a dimethicone.

It is also disclosed the combination (C) comprises the composition (C1) comprising the aforementioned mixture (M1a) and, furthermore, the combination (C) comprises the composition (C2) comprising the aforementioned mixture (M2a),
wherein said combination (C) is for use in a method for the preventive and/or curative treatment of symptoms or diseases relating to or deriving from lacrimal dysfunction syndrome (LDS) or dry eye syndrome, and wherein said combination (C) is for use in a method for treatment in subjects with Reflux Symptom Index (RSI) greater than 13.

It is also disclosed the combination (C) comprises the composition (C1) comprising the aforementioned mixture (M1a) and, furthermore, the combination (C) comprises the composition (C2) comprising the aforementioned mixture (M2a),
wherein said combination (C) is for use in a method for the preventive and/or curative treatment of symptoms or diseases relating to or deriving from lacrimal dysfunction syndrome (LDS) or dry eye syndrome, and wherein said combination (C) is for use in a method for treatment in subjects with Reflux Symptom Index (RSI) greater than 13 and Ocular Surface Disease Index (OSDI) greater than 12; preferably with Ocular Surface Disease Index (OSDI) greater than 22; more preferably with Ocular Surface Disease Index (OSDI) greater than 32.

In a preferred embodiment, the combination (C) comprises:
(I) the composition (C1 ) in form of ophthalmic solution (eye wash) having the following composition in by weight % 500 g of total weight of composition (C1 ):

| Component | % by w/w | Weight (g) |
|---|---|---|
| - Magnesium alginate | 0.20 | 1 |
| - Hyaluronic acid | 0.15 | 0.75 |
| - Glyceric extract of green tea ( *Camellia senensis*) | 1.00 | 5 |
| and, optionally, additives/excipients such as: | | |
| - Sodium tetraborate | 0.20 | 1 |
| - Boric acid | 0.70 | 3.5 |
| - Sodium chloride | 0.24 | 1.2 |
| - Water | 97.51 | 487.55; and |

(II) the composition (C2) having the following formula per 100 g of (C2):

| Component | % by w/w | Weight (g) |
|---|---|---|
| - Sorbitol: | 0.4 | 40 |
| - Magnesium alginate: | 0.17 | 17 |
| - Mannitol: | 0.14 | 14 |
| - Calcium phosphate dibasic anhydrous: | 0.05 | 5 |
| - Potassium bicarbonate: | 0.05 | 5 |
| - Flavour: | 0.05 | 5 |
| - Silicon dioxide: | 0.04 | 4 |
| - Corn starch: | 0.044 | 4.4 |
| - Simethicone: | 0.035 | 3.5 |
| Vegetable magnesium stearate | 0.02 | 2 |
| - Sucralose: | 0.001 | 0.1 |

Furthermore, forming an object of the present invention is a kit comprising said combination (C) for use according to the attached claims, wherein said kit comprises at least one housing in which the composition (C1) in form of eye wash is received and in which the composition (C2) in form of tablet is received.

Preferably, the kit comprises or, alternatively, consists of a secondary packaging receiving the composition (Cl) and the composition (C2). More preferably, the composition (C1) and the composition (C2) are each received in a separate primary packaging - directly at contact with the composition (C1) and with the composition (C2) - the two primary packagings being received in the secondary packaging.

According to an embodiment, the kit comprises a single housing in which the composition (C1) in form of eye wash is received and in which the composition (C2) in form of tablet is received, preferably in which the two packagings are received.

According to another embodiment, the kit comprises:
- a first housing in which the composition (C1) in eye wash is received, preferably the primary packaging of the composition (C1);
- a second housing, separated from said first housing, in which the composition (C2) in form of tablet is received, preferably the primary packaging of the composition (C2).

Unless specified otherwise, the indication that a composition "comprises" one or more components means that other components besides the one, or the ones, indicated specifically, are present even though not necessarily indicated, meaning that the composition can also exclusively contain such components, and the indication that a composition "consists" of determined components means that the presence of other composition is excluded.

In the present invention, the expression "treatment" of a symptom or disease is used to indicate the therapy aimed at restoring a subject's health conditions, maintaining the current conditions and/or preventing the deterioration of said health conditions.

In the present invention, the expression "prevention" of a disease is used to indicate a therapy aimed at hindering the occurrence of such symptom or disease in a subject, including but not exclusively complication or effect of a pre-existing disease.

Unless indicated otherwise, in the present invention the percentages and amounts of a component in a mixture shall be deemed to refer to the weight of such component with respect to the total weight of the mixture.

Unless indicated otherwise, in the present invention, as regards the value intervals of numerical values for a given characteristic, the indication "from X to Y" comprises the extremities, i.e. X and Y, as well as all possible intermediate numerical values.

The expression "medical device" in the context of the present invention is used according to the meaning laid down by the Italian Legislative Decree n° 46, dated 24 February 1997, corresponding to the definition provided by the World Health Organisation and available at http://www.who.int/medical_devices/full_deffinition/en/, i.e. it indicates a substance or another product, used alone or in combination, designated by the manufacturer to be used in humans for diagnosis, prevention, control, therapy or disease attenuation purposes, the product not exercising the main action, in or on the human body, for which it is designated, neither with pharmacological or immunology means nor by means of a metabolic process but the function thereof can be assisted by such means.

Both the mixtures and the compositions subject of the present invention are prepared using techniques, methods and equipment known to the man skilled in the art.

### ADVANTAGES

It was observed that through the combination according to the invention it is possible to obtain a synergic effect of the composition (C1) with the composition (C2) for the preventive and/or curative treatment of symptoms or diseases directly or indirectly relating to or deriving from extraoesophageal reflux or laryngopharyngeal reflux or, generally, from the backflow of the gastric content or acid gastric vapours from the stomach in at least one extraoesophageal area, in subjects suffering from gastroesophageal reflux or in subjects not suffering from gastroesophageal reflux, preferably in subjects suffering from gastroesophageal reflux.

### EXPERIMENTAL PART

The following examples provide practical embodiments of the invention, without limiting the scope of protection thereof in any manner whatsoever.

COMPOSITION 1, ophthalmic solution (eye wash) comprising in by weight % per 500 g of total weight:

| Component | % by w/w | Weight (g) |
|---|---|---|
| - Magnesium alginate | 0.20 | 1 |
| - Hyaluronic acid | 0.15 | 0.75 |
| - Glyceric extract of green tea (*Camellia senensis*) | 1.00 | 5 |
| and, optionally, additives/excipients such as: | | |
| Sodium tetraborate | 0.20 | 1 |
| Boric acid | 0.70 | 3.5 |
| Sodium chloride | 0.24 | 1.2 |
| Water | 97.51 | 487.55 |

COMPOSITION 2, for oral administration (tablet) having the following formula for 100 g:

| Component | % by w/w | Weight (g) |
|---|---|---|
| - Sorbitol: | 0.4 | 40 |
| - Magnesium alginate: | 0.17 | 17 |
| - Mannitol: | 0.14 | 14 |
| - Calcium phosphate dibasic anhydrous: | 0.05 | 5 |
| - Potassium bicarbonate: | 0.05 | 5 |
| - Flavour: | 0.05 | 5 |
| - Silicon dioxide: | 0.04 | 4 |
| - Corn starch: | 0.044 | 4.4 |
| - Simethicone: | 0.035 | 3.5 |
| Vegetable magnesium stearate | 0.02 | 2 |
| - Sucralose: | 0.001 | 0.1 |

## Claims

1. A combination (C) comprising:
(I) a composition (C1) in form of eye wash comprising:
(i) a mixture (M1) comprising, or alternatively, consisting of:
(i-a) an alginic acid, or a salt thereof,
(i-b) a hyaluronic acid, or a salt thereof, and, optionally,
(ii) at least one technological additive and/or at least one pharmaceutical or food grade excipient; and
(II) a composition (C2) in form of tablet comprising:
(iii) a mixture (M2) comprising, or alternatively, consisting of:
(iii-a) an alginic acid or a salt thereof of an alkaline metal or of an alkaline earth metal;
(iii-b) a bicarbonate salt of an alkaline metal or alkaline earth and/or a carbonate salt of an alkaline metal or alkaline earth;
(iii-c) a simethicone and/or dimethicone; and, optionally,
(iv) at least one technological additive and/or at least one pharmaceutical or food grade excipient;
said combination (C) being for use in a method for the preventive or curative treatment of symptoms or diseases of the eyeball and periocular area, wherein said symptoms or diseases relate to, or derive from, extraoesophageal reflux or laryngopharyngeal reflux (LPR) or from the backflow of the gastric content or of the acid gastric vapours from the stomach into at least one extraoesophageal area.

2. The combination (C) for use according to claim 1, wherein said combination (C) is for use in a method for the preventive or curative treatment of the lacrimal dysfunction syndrome (LDS) or dry eye syndrome, conjunctivitis of the conjunctiva, conjunctivitis of the cornea, ocular inflammation or periocular inflammation; wherein said symptoms or diseases relate to, or derive from, extraoesophageal reflux or laryngopharyngeal reflux (LPR) or from the backflow of the gastric content or of the acid gastric vapours from the stomach in said at least one extraoesophageal area.

3. The combination (C) for use according to any one of the preceding claims, wherein said combination (C) is for use in a method for treatment in subjects with Reflux Symptom Index (RSI) greater than 13.

4. The combination (C) for use according to claims 2 and 3, wherein said combination (C) is for use in a method for treatment in subjects with Reflux Symptom Index (RSI) greater than 13 and Ocular Surface Disease Index (OSDI) greater than 12; preferably with Ocular Surface Disease Index (OSDI) greater than 22; more preferably with Ocular Surface Disease Index (OSDI) greater than 32.

5. The combination (C) for use according to claim 3 or 4, wherein said combination (C) is for use in a method for the preventive or curative treatment of symptoms or diseases relating to, or deriving from, the lacrimal dysfunction syndrome (LDS) or dry eye syndrome, and wherein said combination (C) is for use in a method for treatment in subjects with Reflux Symptom Index (RSI) greater than 13; preferably in subjects with Reflux Symptom Index (RSI) greater than 13 and Ocular Surface Disease Index (OSDI) greater than 12 or greater than 22 or greater than 32.

6. The combination (C) for use according to any one of the preceding claims, wherein the composition (C1) is administered topically and wherein the composition (C2) is administered enterally; preferably wherein the composition (C1) is administered topically on the eyeball surface and/or periocular area and wherein the composition (C2) is administered enterally; preferably wherein the composition (C1) is administered topically on an eyeball surface and/or periocular area and wherein the composition (C2) is administered orally.

7. The combination (C) for use according to any one of the preceding claims, wherein said combination (C) is for use in a method for treatment as co-adjuvant for the re-epithelialization processes of at least one mucosa in said at least one extraoesophageal area; preferably the eyeball mucosa or the periocular area mucosa.

8. The combination (C) for use according to any one of the preceding claims, wherein said combination (C) is for use in a method for the simultaneous preventive or curative treatment of at least one first symptom or disease and of at least one second symptom or disease, wherein said at least one first symptom or disease and said at least one second symptom or disease relate to, or derive from, extraoesophageal reflux or laryngopharyngeal reflux (LPR) or from the backflow of the gastric content or acid gastric vapours from the stomach in an extraoesophageal region, wherein said at least one first symptom or disease is a symptom or disease manifested in the eyeball or periocular area, and wherein said at least one second symptom or disease is not a symptom or disease manifested in the eyeball or periocular area.

9. The combination (C) for use according to any one of the preceding claims, wherein in said mixture (M1) comprised in said composition (C1):
- (i-a) is comprised between 0.05% and 5% by weight, preferably comprised between 0.2% and 2% by weight, even more preferably comprised between 0.4% and 0.8% by weight, with respect to the total weight of the composition (C1); and
- (i-b) is comprised between 0.01% and 1% by weight, preferably comprised between 0.05% and 0.5% by weight, even more preferably comprised between 0.15% and 0.30% by weight, with respect to the total weight of the composition (C1).

10. The combination (C) for use according to any one of the preceding claims, wherein in said mixture (M1) comprised in said composition (C1):
- (i-a) is an alginic acid or a salt thereof having an average molecular weight comprised between 50 kDa and 800 kDa, preferably comprised between 100 kDa and 600 kDa, even more preferably comprised between 200 kDa and 400 kDa; and (i-a) is an alginic acid or a salt thereof obtained from marine algae; preferably (i-a) is a salt of an alginic acid, more preferably (i-a) is magnesium alginate; and/or
- (i-b) is a linear or branched hyaluronic acid having an average molecular weight comprised between 200 kDa and 5.000 kDa, preferably comprised between 1.000 kDa and 3.000 kDa; more preferably comprised between 1.500 kDa and 2.000 kDa; preferably (i-b) is a hyaluronic acid salt or, more preferably (i-b) is sodium hyaluronate.

11. The combination (C) for use according to any one of the preceding claims, wherein the mixture (M1) further comprises at least one third compound (i-c) selected from among the group comprising or, alternatively consisting of:
- (i-c1) extract of green tea *(Camellia sinensis),* preferably glyceric extract of green tea at an amount comprised between 0.1% and 5% by weight, more preferably comprised between 0.5% and 3.5% by weight, even more preferably comprised between 1% and 2% by weight, with respect to the weight of the composition (C1);
- (i-c2) rosmarinic acid or 3-(3,4-dihydroxyphenyl)-2R-{[3-(3,4-dihydroxyphenyl)prop-2E-enoyl]oxy}propanoic acid, preferably at an amount comprised between 0.01% and 3% by weight, more preferably comprised between 0.1% and 2% by weight, even more preferably comprised between 0.5% and 1% by weight, with respect to the weight of the composition (C1);
- (i-c3) ethylenediaminetetraacetic acid (EDTA) or a salt thereof, preferably at an amount comprised between 0.05% and 0.3% by weight, more preferably comprised between 0.1% and 0.25% by weight, even more preferably comprised between 0.15% and 0.20% by weight, with respect to the weight of the composition (C1);
- and the mixtures thereof.

12. The combination (C) for use according to any one of the preceding claims, wherein in said mixture (M2) comprised in said composition (C2):
- (iii-a) is comprised between 20% and 70% by weight; preferably comprised between 40% and 60% by weight, with respect to the total weight of the mixture (M2),
- (iii-b) is comprised between 5% and 30% by weight; preferably comprised between 10% and 25% by weight, with respect to the total weight of the mixture (M2), and
- (iii-c) is comprised between 5% and 30% by weight; preferably comprised between 10% and 25% by weight, with respect to the total weight of the mixture (M2).

13. The combination (C) for use according to any one of the preceding claims, wherein in said mixture (M2) comprised in said composition (C2):
- (iii-a) is an alginic acid or a salt thereof of an alkaline metal or of an alkaline earth metal having an average molecular weight comprised between about 50 KDa and about 1000 KDa, preferably comprised between about 100 KDa and about 800 KDa, more preferably comprised between about 200 KDa and about 400 KDa; more preferably it has an average molecular weight of about 240 kDa or an atomic mass unit; and (iii-a) is an alginic acid or a salt thereof of an alkaline metal or of an alkaline earth metal obtained from marine algae; preferably (iii-a) is an alginic acid salt, more preferably said alginic acid salt is selected from among the group comprising or, alternatively, consisting of: sodium alginate, potassium alginate, calcium alginate and magnesium alginate, more preferably (iii-a) is magnesium alginate; and/or
- (iii-b) is a bicarbonate salt of an alkaline metal or of an alkaline earth metal selected from among the group comprising or, alternatively, consisting of: sodium bicarbonate, potassium bicarbonate, magnesium bicarbonate and calcium bicarbonate, and/or a carbonate salt of an alkaline metal or of an alkaline earth metal selected from among the group comprising or, alternatively, consisting of: sodium carbonate, potassium carbonate, magnesium carbonate and calcium carbonate; more preferably (iii-b) is potassium bicarbonate; and/or
- (iii-c) is a simethicone selected from among the group comprising or, alternatively, consisting of: Simethicone 100, Simethicone Emulsion 30%, Simethicone Emulsion, Antifoam C100 (or C100F), Antifoam 30PD, Simethicone Antifoam PD30, Simethicone Antifoam 30 MG; and/or a dimethicone selected from among the group comprising or, alternatively, consisting of: Cetyl dimethicone, Stearyl dimethicone, Stearoxy dimethicone, Behenoxy dimethicone and all polymers belonging to the group of dimethicone copolyols.

14. The combination (C) for use according to any one of the preceding claims, wherein said mixture (M1) comprises or, alternatively, consists of:
- (i-a) magnesium alginate,
- (i-b) sodium hyaluronate, and
- (i-c) extract of green tea or *Camellia sinensis*; and
wherein said mixture (M2) comprises or, alternatively, consists of:
- (iii-a) magnesium alginate;
- (iii-b) potassium bicarbonate;
- (iii-c) simethicone and/or dimethicone.

15. A kit comprising the combination (C) for use according to any one of the preceding claims, wherein said kit comprises at least one housing in which the composition (C1) in form of eye wash is received and in which the composition (C2) in form of tablet is received;
said kit preferably comprising:
- a first housing in which the composition (C1) in eye wash is received;
- a second housing, separated from said first housing, in which the composition (C2) in form of tablet is received.

## Patentansprüche

1. Kombination (C), umfassend:
(I) eine Zusammensetzung (C1) in Form einer Augenspülung, umfassend:
(i) eine Mischung (M1), umfassend oder alternativ bestehend aus:
(i-a) eine/r Alginsäure oder ein/em Salz davon,
(i-b) eine/r Hyaluronsäure oder ein/em Salz davon und gegebenenfalls,
(ii) mindestens einen/m technologischen Zusatzstoff und/oder mindestens einen/m pharmazeutischen oder lebensmitteltauglichen Hilfsstoff; und
(II) eine Zusammensetzung (C2) in Form einer Tablette, umfassend:
(iii) eine Mischung (M2), umfassend oder alternativ bestehend aus:
(iii-a) eine/r Alginsäure oder ein/em Salz davon eines Alkalimetalls oder eines Erdalkalimetalls;
(iii-b) ein/em Bicarbonatsalz eines Alkalimetalls oder einer Erdalkalimetallverbindung und/oder ein/em Carbonatsalz eines Alkalimetalls oder einer Erdalkalimetallverbindung;
(iii-c) ein/em Simethicon und/oder Dimethicon; und gegebenenfalls
(iv) mindestens einen/m technologischen Zusatzstoff und/oder mindestens einen/m pharmazeutischen oder lebensmitteltauglichen/m Hilfsstoff;
wobei die Kombination (C) zur Verwendung in einem Verfahren zur präventiven oder kurativen Behandlung von Symptomen oder Erkrankungen des Augapfels und des periokularen Bereichs bestimmt ist, wobei sich die Symptome oder Erkrankungen auf extraösophagealen Reflux oder laryngopharyngealen Reflux (LPR) oder auf den Rückfluss des Mageninhalts oder der sauren Magendämpfe aus dem Magen in mindestens einen extraösophagealen Bereich beziehen oder davon herrühren.

2. Kombination (C) zur Verwendung nach Anspruch 1, wobei die Kombination (C) zur Verwendung in einem Verfahren zur präventiven oder kurativen Behandlung des Tränendysfunktionssyndroms (LDS) oder des Syndroms des trockenen Auges, der Konjunktivitis der Bindehaut, der Konjunktivitis der Hornhaut, der Augenentzündung oder der periokularen Entzündung bestimmt ist; wobei sich die Symptome oder Erkrankungen auf extraösophagealen Reflux oder laryngopharyngealen Reflux (LPR) oder auf den Rückfluss des Mageninhalts oder der sauren Magendämpfe aus dem Magen in dem mindestens einen extraösophagealen Bereich beziehen oder davon herrühren.

3. Kombination (C) zur Verwendung nach einem der vorhergehenden Ansprüche, wobei die Kombination (C) zur Verwendung in einem Verfahren zur Behandlung von Patienten mit einem Reflux-Symptom-Index (RSI) größer als 13 bestimmt ist.

4. Kombination (C) zur Verwendung nach den Ansprüchen 2 und 3, wobei die Kombination (C) zur Verwendung in einem Verfahren zur Behandlung von Patienten mit einem Reflux-Symptom-Index (RSI) größer als 13 und einem Index für Erkrankungen der Augenoberfläche (OSDI) größer als 12 bestimmt ist; vorzugsweise mit einem Index für Erkrankungen der Augenoberfläche (OSDI) größer als 22; bevorzugter mit einem Index für Erkrankungen der Augenoberfläche (OSDI) größer als 32.

5. Kombination (C) zur Verwendung nach Anspruch 3 oder 4, wobei die Kombination (C) zur Verwendung in einem Verfahren zur präventiven oder kurativen Behandlung von Symptomen oder Erkrankungen bestimmt ist, die sich auf das Tränendysfunktionssyndrom (LDS) oder das Syndrom des trockenen Auges beziehen oder davon herrühren, und wobei die Kombination (C) zur Verwendung in einem Verfahren zur Behandlung bei Patienten mit einem Reflux-Symptom-Index (RSI) größer als 13 bestimmt ist; vorzugsweise bei Patienten mit einem Reflux-Symptom-Index (RSI) größer als 13 und einem Index für Erkrankungen der Augenoberfläche (OSDI) größer als 12 oder größer als 22 oder größer als 32.

6. Kombination (C) zur Verwendung nach einem der vorhergehenden Ansprüche, wobei die Zusammensetzung (C1) topisch verabreicht wird und wobei die Zusammensetzung (C2) enteral verabreicht wird; vorzugsweise wobei die Zusammensetzung (C1) topisch auf der Augapfeloberfläche und/oder dem periokularen Bereich verabreicht wird und wobei die Zusammensetzung (C2) enteral verabreicht wird; vorzugsweise wobei die Zusammensetzung (C1) topisch auf einer Augapfeloberfläche und/oder dem periokularen Bereich verabreicht wird und wobei die Zusammensetzung (C2) oral verabreicht wird.

7. Kombination (C) zur Verwendung nach einem der vorhergehenden Ansprüche, wobei die Kombination (C) zur Verwendung in einem Verfahren zur Behandlung als Co-Adjuvans für die Reepithelisierungsprozesse von mindestens einer Schleimhaut in dem mindestens einen extraösophagealen Bereich, vorzugsweise der Augapfelschleimhaut oder den Schleimhäuten des periokularen Bereichs, bestimmt ist.

8. Kombination (C) zur Verwendung nach einem der vorhergehenden Ansprüche, wobei die Kombination (C) zur Verwendung in einem Verfahren zur gleichzeitigen präventiven oder kurativen Behandlung von mindestens einem ersten Symptom oder einer Erkrankung und von mindestens einem zweiten Symptom oder einer Erkrankung bestimmt ist, wobei sich das mindestens eine erste Symptom oder die Erkrankung und das mindestens eine zweite Symptom oder die Erkrankung auf extraösophagealen Reflux oder laryngopharyngealen Reflux (LPR) oder auf den Rückfluss des Mageninhalts oder saurer Magendämpfe aus dem Magen in einer extraösophagealen Region beziehen oder davon herrühren, wobei das/die mindestens eine erste Symptom oder Erkrankung ein Symptom oder eine Erkrankung ist, die sich im Augapfel oder periokularen Bereich manifestiert, und wobei das/die mindestens eine zweite Symptom oder Erkrankung kein Symptom oder keine Erkrankung ist, die sich im Augapfel oder periokularen Bereich manifestiert.

9. Kombination (C) zur Verwendung nach einem der vorhergehenden Ansprüche, wobei in der Mischung (M1), die in der Zusammensetzung (C1) enthalten ist:
- (i-a) zwischen 0,05 und 5 Gew.- % ist, vorzugsweise zwischen 0,2 und 2 Gew.- %, noch bevorzugter zwischen 0,4 und 0,8 Gew.- %, bezogen auf das Gesamtgewicht der Zusammensetzung (C1); und
- (i-b) ist zwischen 0,01 und 1 Gew.- %, vorzugsweise zwischen 0,05 und 0,5 Gew.- %, noch bevorzugter zwischen 0,15 und 0,30 Gew.- %, bezogen auf das Gesamtgewicht der Zusammensetzung (C1).

10. Kombination (C) zur Verwendung nach einem der vorhergehenden Ansprüche, wobei in der Mischung (M1), die in der Zusammensetzung (C1) enthalten ist:
- (i-a) eine Alginsäure oder ein Salz davon mit einem mittleren Molekulargewicht zwischen 50 kDa und 800 kDa ist, vorzugsweise zwischen 100 kDa und 600 kDa, noch bevorzugter zwischen 200 kDa und 400 kDa ist; und (i-a) ist eine Alginsäure oder ein Salz davon, die/das aus Meeresalgen gewonnen wird; vorzugsweise ist (i-a) ein Salz einer Alginsäure, noch bevorzugter ist (i-a) Magnesiumalginat; und/oder
- (i-b) ist eine lineare oder verzweigte Hyaluronsäure mit einem mittleren Molekulargewicht zwischen 200 kDa und 5.000 kDa, vorzugsweise zwischen 1.000 kDa und 3.000 kDa; bevorzugter zwischen 1.500 kDa und 2.000 kDa; vorzugsweise ist (i-b) ein Hyaluronsäuresalz oder, bevorzugter ist (i-b) Natriumhyaluronat.

11. Kombination (C) zur Verwendung nach einem der vorhergehenden Ansprüche, wobei die Mischung (M1) ferner mindestens eine dritte Verbindung (i-c) umfasst, ausgewählt aus der Gruppe umfassend oder alternativ bestehend aus:
- (i-c1) Extrakt aus grünem Tee *(Camellia sinensis),* vorzugsweise Glycerin-Extrakt aus grünem Tee in einer Menge zwischen 0,1 und 5 Gew.- %, bevorzugter zwischen 0,5 und 3,5 Gew.- %, noch bevorzugter zwischen 1 und 2 Gew.- %, bezogen auf das Gewicht der Zusammensetzung (C1);
- (i-c2) Rosmarinsäure oder 3-(3,4-Dihydroxyphenyl)-2R-{[3-(3,4-dihydroxyphenyl)prop-2E-enoyl]oxy}propansäure, vorzugsweise in einer Menge zwischen 0,01 und 3 Gew.- %, bevorzugter zwischen 0,1 und 2 Gew.- %, noch bevorzugter zwischen 0,5 und 1 Gew.- %, bezogen auf das Gewicht der Zusammensetzung (C1);
- (i-c3) Ethylendiamintetraessigsäure (EDTA) oder ein Salz davon, vorzugsweise in einer Menge zwischen 0,05 und 0,3 Gew.- %, bevorzugter zwischen 0,1 und 0,25 Gew.- %, noch bevorzugter zwischen 0,15 und 0,20 Gew.- %, bezogen auf das Gewicht der Zusammensetzung (C1);
- sowie Mischungen hiervon.

12. Kombination (C) zur Verwendung nach einem der vorhergehenden Ansprüche, wobei in der Mischung (M2), die in der Zusammensetzung (C2) enthalten ist:
- (iii-a) zwischen 20 und 70 Gew.- % ist; vorzugsweise zwischen 40 und 60 Gew.- %, bezogen auf das Gesamtgewicht der Mischung (M2),
- (iii-b) zwischen 5 und 30 Gew.- % ist, vorzugsweise zwischen 10 und 25 Gew.- %, bezogen auf das Gesamtgewicht der Mischung (M2), und
- (iii-c) zwischen 5 und 30 Gew.- % ist, vorzugsweise zwischen 10 und 25 Gew.- %, bezogen auf das Gesamtgewicht der Mischung (M2).

13. Kombination (C) zur Verwendung nach einem der vorhergehenden Ansprüche, wobei in der Mischung (M2), die in der Zusammensetzung (C2) enthalten ist:
- (iii-a) eine Alginsäure oder ein Salz davon eines Alkalimetalls oder eines Erdalkalimetalls mit einem mittleren Molekulargewicht zwischen etwa 50 KDa und etwa 1000 KDa ist, vorzugsweise zwischen etwa 100 KDa und etwa 800 KDa, bevorzugter zwischen etwa 200 KDa und etwa 400 KDa; bevorzugter weist es ein mittleres Molekulargewicht von etwa 240 kDa oder einer Atommasseneinheit auf; und (iii-a) ist eine Alginsäure oder ein Salz davon eines Alkalimetalls oder eines Erdalkalimetalls, das aus Meeresalgen gewonnen wird; bevorzugt ist (iii-a) ein Alginsäuresalz, bevorzugter ist das Alginsäuresalz ausgewählt aus der Gruppe umfassend oder alternativ bestehend aus: Natriumalginat, Kaliumalginat, Calciumalginat und Magnesiumalginat, bevorzugter ist (iii-a) Magnesiumalginat; und/oder
- (iii-b) ein Bicarbonatsalz eines Alkalimetalls oder eines Erdalkalimetalls ist, ausgewählt aus der Gruppe umfassend oder alternativ bestehend aus: Natriumbicarbonat, Kaliumbicarbonat, Magnesiumbicarbonat und Calciumbicarbonat, und/oder ein Carbonatsalz eines Alkalimetalls oder eines Erdalkalimetalls, ausgewählt aus der Gruppe umfassend oder alternativ bestehend aus: Natriumcarbonat, Kaliumcarbonat, Magnesiumcarbonat und Calciumcarbonat; bevorzugter ist (iii-b) Kaliumbicarbonat; und/oder
- (iii-c) ein Simethicon ist, ausgewählt aus der Gruppe umfassend oder alternativ bestehend aus: Simethicone 100, Simethicone Emulsion 30%, Simethicone Emulsion, Antifoam C100 (oder C100F), Antifoam 30PD, Simethicone Antifoam PD30, Simethicone Antifoam 30 MG; und/oder ein/em Dimethicone ausgewählt aus der Gruppe umfassend oder alternativ bestehend aus: Cetyldimethicon, Stearyldimethicon, Stearoxydimethicon, Behenoxydimethicon und alle/n Polymere, die zur Gruppe der Dimethiconcopolyole gehören.

14. Kombination (C) zur Verwendung nach einem der vorhergehenden Ansprüche, wobei die Mischung (M1) umfasst oder alternativ besteht aus:
- (i-a) Magnesiumalginat,
- (i-b) Natriumhyaluronat, und
- (i-c) Extrakt aus grünem Tee oder *Camellia sinensis*; und
wobei die Mischung (M2) umfasst oder alternativ besteht aus:
- (iii-a) Magnesiumalginat;
- (iii-b) Kaliumbicarbonat;
- (iii-c) Simethicon und/oder Dimethicon.

15. Kit, umfassend die Kombination (C) zur Verwendung nach einem der vorhergehenden Ansprüche, wobei der Kit mindestens ein Gehäuse umfasst, in dem die Zusammensetzung (C1) in Form einer Augenspülung aufgenommen wird und in dem die Zusammensetzung (C2) in Form einer Tablette aufgenommen wird; wobei das Kit vorzugsweise Folgendes umfasst:
- ein erstes Gehäuse, in dem die Zusammensetzung (C1) in Augenspülung aufgenommen wird;
- ein zweites Gehäuse, das von dem ersten Gehäuse getrennt ist, in dem die Zusammensetzung (C2) in Form einer Tablette aufgenommen ist.

## Revendications

1. Combinaison (C), comprenant :
(I) une composition (C1) sous forme de lave-oeil, comprenant :
(i) un mélange (M1), comprenant ou, alternativement, constitué :
(i-a) d'un acide alginique ou un de ses sels,
(i-b) d'un acide hyaluronique, ou un de ses sels, et, éventuellement,
(ii) au moins un additif technologique et/ou au moins un excipient de qualité pharmaceutique ou alimentaire ; et
(II) une composition (C2) sous forme de comprimé, comprenant :
(iii) un mélange (M2), comprenant, ou alternativement, constitué :
(iii-a) d'un acide alginique ou un de ses sels d'un métal alcalin ou d'un métal alcalino-terreux ;
(iii-b) d'un sel de bicarbonate d'un métal alcalin ou alcalino-terreux et/ou un sel de carbonate d'un métal alcalin ou alcalino-terreux ;
(iii-c) d'une siméthicone et/ou une diméthicone ; et, éventuellement,
(iv) d'au moins un additif technologique et/ou au moins un excipient de qualité pharmaceutique ou alimentaire ;
ladite combinaison (C) est destinée à être utilisée dans une méthode de traitement préventif ou curatif des symptômes ou des maladies du globe oculaire et de la région périoculaire, dans laquelle lesdits symptômes ou maladies sont liés ou découlent du reflux extra-oesophagien ou du reflux laryngopharyngé (LPR) ou du retour du contenu gastrique ou des vapeurs gastriques acides de l'estomac dans au moins une région extra-oesophagienne.

2. Combinaison (C) destinée à être utilisée selon la revendication 1, dans laquelle ladite combinaison (C) est destinée à être utilisée dans une méthode de traitement préventif ou curatif du syndrome de dysfonctionnement lacrymal (LDS) ou du syndrome de l'œil sec, de la conjonctivite de la conjonctive, de la conjonctivite s'étendant à la cornée, de l'inflammation oculaire ou de l'inflammation périoculaire ; dans laquelle lesdits symptômes ou maladies sont liés ou découlent du reflux extra-oesophagien ou du reflux laryngopharyngé (LPR) ou du retour du contenu gastrique ou des vapeurs gastriques acides de l'estomac dans ladite au moins une zone extra-oesophagienne.

3. Combinaison (C) destinée à être utilisée selon l'une quelconque des revendications précédentes, dans laquelle ladite combinaison (C) est destinée à être utilisée dans une méthode de traitement chez des sujets dont l'indice de reflux (RSI) est supérieur à 13.

4. Combinaison (C) destinée à être utilisée selon les revendications 2 et 3, dans laquelle ladite combinaison (C) est destinée à être utilisée dans une méthode de traitement chez des sujets présentant un indice de reflux (RSI) supérieur à 13 et un indice de maladie de la surface oculaire (OSDI) supérieur à 12; de préférence avec un indice de maladie de la surface oculaire (OSDI) supérieur à 22 ; plus préférablement avec un indice de maladie de la surface oculaire (OSDI) supérieur à 32.

5. Combinaison (C) destinée à être utilisée selon la revendication 3 ou 4, dans laquelle ladite combinaison (C) est destinée à être utilisée dans une méthode de traitement préventif ou curatif des symptômes ou maladies liés au, ou dérivant du, syndrome de dysfonctionnement lacrymal (SDL) ou syndrome de l'œil sec, et dans laquelle ladite combinaison (C) est destinée à être utilisée dans une méthode de traitement chez les sujets présentant un indice de reflux (RSI) supérieur à 13 ; de préférence chez les sujets dont l'indice de reflux (RSI) est supérieur à 13 et dont l'indice de maladie de la surface oculaire (OSDI) est supérieur à 12 ou supérieur à 22 ou supérieur à 32.

6. Combinaison (C) destinée à être utilisée selon l'une quelconque des revendications précédentes, dans laquelle la composition (C1) est administrée par voie topique et dans laquelle la composition (C2) est administrée par voie entérale ; de préférence, dans laquelle la composition (C1) est administrée par voie topique sur la surface du globe oculaire et/ou la zone périoculaire et dans laquelle la composition (C2) est administrée par voie entérale ; de préférence, dans laquelle la composition (C1) est administrée par voie topique sur la surface du globe oculaire et/ou la zone périoculaire et dans laquelle la composition (C2) est administrée par voie orale.

7. Combinaison (C) destinée à être utilisée selon l'une quelconque des revendications précédentes, dans laquelle ladite combinaison (C) est destinée à être utilisée dans une méthode de traitement comme coadjuvant pour les processus de réépithélialisation d'au moins une muqueuse dans ladite au moins une zone extra-oesophagienne ; de préférence la muqueuse du globe oculaire ou la muqueuse de la zone périoculaire.

8. Combinaison (C) destinée à être utilisée selon l'une quelconque des revendications précédentes, dans laquelle ladite combinaison (C) est destinée à être utilisée dans une méthode pour le traitement préventif /ou curatif simultané d'au moins un premier symptôme ou maladie et d'au moins un second symptôme ou maladie, dans laquelle ledit au moins un premier symptôme ou maladie et ledit au moins un second symptôme ou maladie sont liés à, ou dérivent du reflux extra-oesophagien ou du reflux laryngopharyngé (LPR) ou du retour du contenu gastrique ou des vapeurs gastriques acides de l'estomac dans une région extra-oesophagienne, dans laquelle ledit au moins un premier symptôme ou maladie est un symptôme ou une maladie se manifestant dans le globe oculaire ou la région périoculaire, et dans laquelle ledit au moins un second symptôme ou maladie n'est pas un symptôme ou une maladie se manifestant dans le globe oculaire ou la région périoculaire.

9. Combinaison (C) destinée à être utilisée selon l'une quelconque des revendications précédentes, dans laquelle dans ledit mélange (M1) compris dans ladite composition (C1) :
- (i-a) est compris entre 0,05 et 5 % en poids, de préférence compris entre 0,2 et 2 % en poids, encore plus préférablement compris entre 0,4 et 0,8 % en poids, par rapport au poids total de la composition (C1) ; et
- (i-b) est compris entre 0,01 et 1 % en poids, de préférence compris entre 0,05 et 0,5 % en poids, encore plus préférablement compris entre 0,15 et 0,30 % en poids, par rapport au poids total de la composition (C1).

10. Combinaison (C) destinée à être utilisée selon l'une quelconque des revendications précédentes, dans laquelle dans ledit mélange (M1) compris dans ladite composition (C1) :
- (i-a) est un acide alginique ou un sel de cet acide ayant un poids moléculaire moyen compris entre 50 et 800 kDa, de préférence compris entre 100 et 600 kDa, encore plus préférablement compris entre 200 et 400 kDa ; et (i-a) est un acide alginique ou un sel de cet acide obtenu à partir d'algues marines ; de préférence (i-a) est un sel d'un acide alginique, plus préférablement (i-a) est l'alginate de magnésium ; et/ou
- (i-b) est un acide hyaluronique linéaire ou ramifié ayant un poids moléculaire moyen compris entre 200 et 5 000 kDa, de préférence compris entre 1 000 et 3 000 kDa ; plus préférablement compris entre 1 500 et 2000 kDa ; de préférence i-b) est un sel d'acide hyaluronique ou, plus préférablement (i-b) est un hyaluronate de sodium.

11. Combinaison (C) destinée à être utilisée selon l'une quelconque des revendications précédentes, dans laquelle le mélange (M1) comprend de plus au moins un troisième composé (i-c) choisi dans le groupe comprenant ou, alternativement, constitué :
- (i-c1) d'un extrait de thé vert *(Camellia sinensis),* de préférence un extrait glycérique de thé vert dans une quantité comprise entre 0,1 et 5 % en poids, plus préférablement comprise entre 0,5 et 3,5 % en poids, encore plus préférablement comprise entre 1 et 2 % en poids, par rapport au poids de la composition (C1) ;
- (i-c2) d'un acide rosmarinique ou acide 3-(3,4-dihydroxyphényl)-2R-{[3-(3,4-dihydroxyphényl)prop-2E-énoyl]oxy}propionique, de préférence dans une quantité comprise entre 0,01 et 3 % en poids, plus préférablement comprise entre 0,1 et 2 % en poids, encore plus préférablement comprise entre 0,5 et 1 % en poids, par rapport au poids de la composition (C1) ;
- (i-c3) d'un acide éthylène diamine tétra acétique (EDTA) ou un de ses sels, de préférence dans une quantité comprise entre 0,05 et 0,3 % en poids, plus préférablement comprise entre 0,1 et 0,25 % en poids, encore plus préférablement comprise entre 0,15 et 0,20 % en poids, par rapport au poids de la composition (C1);
- et leurs mélanges.

12. Combinaison (C) destinée à être utilisée selon l'une quelconque des revendications précédentes, dans laquelle dans ledit mélange (M2) compris dans ladite composition (C2) :
- (iii-a) est compris entre 20 et 70 % en poids ; de préférence compris entre 40 et 60 % en poids, par rapport au poids total du mélange (M2),
- (iii-b) est compris entre 5 et 30 % en poids ; de préférence compris entre 10 et 25 % en poids, par rapport au poids total du mélange (M2), et
- (iii-c) est comprise entre 5 et 30 % en poids ; de préférence comprise entre 10 et 25 % en poids, par rapport au poids total du mélange (M2).

13. Combinaison (C) destinée à être utilisée selon l'une quelconque des revendications précédentes, dans laquelle dans ledit mélange (M2) compris dans ladite composition (C2) :
- (iii-a) est un acide alginique ou un de ses sels d'un métal alcalin ou d'un métal alcalino-terreux ayant un poids moléculaire moyen compris entre environ 50 et environ 1 000 KDa, de préférence compris entre environ 100 et environ 800 KDa, plus préférablement compris entre environ 200 et environ 400 KDa ; plus préférablement, il a un poids moléculaire moyen d'environ 240 kDa ou une unité de masse atomique ; et (iii-a) est un acide alginique ou un de ses sels d'un métal alcalin ou d'un métal alcalino-terreux obtenu à partir d'algues marines ; de préférence (iii-a) est un sel d'acide alginique, plus préférablement ledit sel d'acide alginique est choisi dans le groupe comprenant ou, alternativement, constitué : d'alginate de sodium, d'alginate de potassium, d'alginate de calcium et d'alginate de magnésium, plus préférablement (iii-a) est de l'alginate de magnésium ; et/ou
- (iii-b) est un sel de bicarbonate d'un métal alcalin ou d'un métal alcalino-terreux choisi dans le groupe comprenant ou, alternativement, constitué de : bicarbonate de sodium, bicarbonate de potassium, bicarbonate de magnésium et bicarbonate de calcium, et/ou un sel de carbonate d'un métal alcalin ou d'un métal alcalino-terreux choisi dans le groupe comprenant ou, alternativement, constitué de : carbonate de sodium, carbonate de potassium, carbonate de magnésium et de carbonate de calcium ; plus préférablement, (iii-b) est le bicarbonate de potassium ; et/ou
- (iii-c) est une siméthicone choisie dans le groupe comprenant ou, alternativement, constitué de : Siméthicone 100, d'une émulsion de Siméthicone 30 %, d'une émulsion de Siméthicone, d'un agent antimousse C100 (ou C100F), d'un agent antimousse 30PD, d'un agent antimousse de Siméthicone PD30, d'un agent antimousse de Siméthicone 30 MG ; et/ou une diméthicone choisie dans le groupe comprenant ou, alternativement, constitué de : Cétyl diméthicone, Stéaryl diméthicone, Stéaroxy diméthicone, Behenoxy diméthicone et de tous les polymères appartenant au groupe des copolyols de diméthicone.

14. Combinaison (C) destinée à être utilisée selon l'une quelconque des revendications précédentes, dans laquelle ledit mélange (M1) comprend ou, alternativement, est constitué :
- (i-a) d'alginate de magnésium,
- (i-b) de hyaluronate de sodium, et
- (i-c) d'extrait de thé vert ou de *Camellia sinensis* ; et
dans laquelle ledit mélange (M2) comprend ou, alternativement, est constitué :
- (iii-a) d'alginate de magnésium ;
- (iii-b) de bicarbonate de potassium ;
- (iii-c) de siméthicone et/ou diméthicone.

15. Kit, comprenant la combinaison (C) destinée à être utilisée selon l'une quelconque des revendications précédentes, dans lequel ledit kit comprend au moins un logement dans lequel est reçue la composition (C1) sous forme de lave-oeil et dans lequel est reçue la composition (C2) sous forme de comprimé ;
ledit kit comprenant de préférence :
- un premier logement dans lequel est reçue la composition (C1) sous forme de lave-oeil ;
- un second logement, séparé dudit premier logement, dans lequel est reçue la composition (C2) sous forme de comprimé.
